Europäisches Patentamt

European Patent Office

Office européen des brevets

(19)

(11) Veröffentlichungsnummer: **0 028 826**
**B1**

(12) **EUROPÄISCHE PATENTSCHRIFT**

(45) Veröffentlichungstag der Patentschrift:
05.12.84

(51) Int. Cl.³: **G 01 N 27/56**

(21) Anmeldenummer: 80106886.7

(22) Anmeldetag: 08.11.80

(54) Elektrochemische Messvorrichtung für die Bestimmung des Sauerstoffgehalts in Gasen und ihre Verwendung.

(30) Priorität: 13.11.79 DE 2945698

(43) Veröffentlichungstag der Anmeldung:
20.05.81 Patentblatt 81/20

(45) Bekanntmachung des Hinweises auf die Patenterteilung:
05.12.84 Patentblatt 84/49

(84) Benannte Vertragsstaaten:
FR GB IT

(56) Entgegenhaltungen:
AT - B - 177 140
DE - A - 1 923 832
DE - A - 1 954 663

(73) Patentinhaber: BROWN, BOVERI & CIE
Aktiengesellschaft Mannheim, Kallstadter Strasse 1,
D-6800 Mannheim Käfertal (DE)

(72) Erfinder: Rohr, Franz-Josef, Dr., Forstweg 2,
D-6941 Abtsteinach (DE)
Erfinder: Krapf, Rudolf, Erlenweg 5, D-6906 Leimen (DE)

(74) Vertreter: Kempe, Wolfgang, Dr. et al, c/o Brown, Boveri
& Cie AG Postfach 351, D-6800 Mannheim 1 (DE)

## Beschreibung

Die Erfindung bezieht sich auf eine elektrochemische Messvorrichtung gemäss dem Oberbegriff des Patentanspruches 1.

Aus der DE-A-1 954 663 ist eine Messvorrichtung bekannt, die einen Festelektrolyten aufweist, der mit zwei Elektroden versehen ist. Zusätzlich weist die Messvorrichtung im Bereich des Festelektrolyten ein Heizelement auf, mit dem die Messvorrichtung auf einer Arbeitstemperatur von 660 bis 1000 °C gehalten wird. An die beiden Elektroden ist eine Gleichspannungsquelle angeschlossen. Die Grösse der Gleichspannung ist so gewählt, dass zwischen den Elektroden nur ein diffusionsbegrenzter Strom fliessen kann. Der zwischen den Elektroden fliessende Strom wird durch die Geschwindigkeit bestimmt, mit der der Sauerstoff aus der Gasprobenmenge in die Oberfläche der Elektrode diffundiert. Somit sind die Diffusionsgeschwindigkeit und der Strom eine Funktion der in der Gasprobe befindlichen Sauerstoffmenge. Der so erhaltene diffusionsbegrenzte Stromzustand macht den Zellenstrom über einen bestimmten Zellenspannungsbereich konstant und unmittelbar von der Geschwindigkeit, mit der der Sauerstoff in die Elektrode diffundiert, abhängig.

Von Nachteil ist hierbei, dass diese Messvorrichtung nur für die Sauerstoffbestimmung von Gasen verwendet werden kann, deren Sauerstoffgehalt relativ klein ist.

Um bei Gasen mit einem grossen Sauerstoffgehalt eine quantitative Aussage über ihren Sauerstoffgehalt machen zu können, ist es erforderlich, den gesamten Sauerstoff aus dem Gasstrom zu extrahieren, die Grösse des Gasstromes zu erfassen und den Gasstrom während des Messvorgangs konstant zu halten. Während die erste Forderung durch ausreichende Grössen der Elektroden, die zweite Forderung durch eine Eichung auf einfache Weise erreicht werden kann, ist die dritte Forderung, nämlich die Konstanthaltung des Gasstromes, meist schwierig und nur mit grossem Aufwand durchzuführen.

Der Erfindung liegt deshalb die Aufgabe zugrunde, eine elektrochemische Messvorrichtung so zu schaffen, dass der Messgasstrom auf einfache Weise, unter Vermeidung eines grossen konstruktiven Aufwandes und zudem kostengünstig in zumindest ausreichendem Masse konstant gehalten werden kann, wobei die Messvorrichtung ausserdem weitgehend wartungsfrei und in hohem Masse betriebssicher sein soll.

Diese Aufgabe wird erfindungsgemäss durch die im Patentanspruch 1 angegebene Messvorrichtung gelöst.

Anspruch 11 hat eine Verwendung dieser Messvorrichtung zum Gegenstand.

Es wird also von dem physikalischen Gesetz Gebrauch gemacht, dass durch eine Verengung oder Drosselstelle, welche einen Zuströmraum mit einem Abströmraum verbindet, das Gas höchstens mit Schallgeschwindigkeit strömen kann, falls ein bestimmtes Druckverhältnis zwischen Abströmraum und Zuströmraum nicht überschritten wird. Die Schallgeschwindigkeit ist hierbei abhängig von den Zustandsgrössen des Gasstroms in der Verengung. Sind nun Druck und Temperatur des Gasstroms im Zuströmraum konstant, so ist auch die Gasgeschwindigkeit in der Verengung konstant und vollkommen unabhängig vom Gegendruck des Gasstroms der Abströmseite, falls eben das bestimmte Druckverhältnis nicht überschritten wird. Dieses Druckverhältnis ist unter der Bezeichnung kritisches Druckverhältnis bekannt, es ist von der Atomzahl der Gase abhängig, beträgt für zweiatomige Gase, also auch für Verbrennungsabgase, 0,528 und kann berechnet werden (vgl. Herning, Stoffströme in Rohrleitungen, 1957, VDI-Verlag Düsseldorf).

Für die Auslegung des Durchtrittsquerschnittes der Verengung ist zu beachten, dass das Gas an der engsten Stelle der Verengung mit Schallgeschwindigkeit strömt, wobei der für die Strömung massgebliche Querschnitt geringer sein kann als der tatsächlich vorhandene Durchtrittsquerschnitt. Dies ist bedingt durch Kontraktion des Gasstromes in der Verengung. Desweiteren kann der tatsächliche Wert für das kritische Druckverhältnis von dem errechneten Wert abweichen, dies gilt insbesondere dann, wenn die Durchströmung der Verengung mit Reibungsverlusten behaftet ist. Bei der Eichung der Messvorrichtung, die zur Bestimmung der Grösse des Gasstroms gegebenenfalls erforderlich ist, lässt sich das kritische Druckverhältnis, ab welchem der Gasstrom konstant ist, leicht feststellen.

Die Verengung könnte an sich beliebig, zum Beispiel in Form einer Kapillare ausgebildet sein, vorteilhaft ist es jedoch, wenn die Verengung die Form einer Blende oder insbesondere einer Düse aufweist. Neben einer einfachen Herstellung ergibt sich in diesem Falle der Vorteil, dass die Reibungsverluste in der Verengung gering sind und somit weitgehende Übereinstimmung mit den vorberechneten Werten für das kritische Druckverhältnis gegeben ist.

Um die Abnutzung der Durchtrittsstelle zu verringern und um Korrosionen zu vermeiden, kann gemäss einer empfehlenswerten Weiterbildung die Blende bzw. Düse aus Glas bestehen.

Der Durchtrittsquerschnitt ist im Einzelfall auf die Grösse des Gasstromes abzustimmen, eine Dimensionierung, die bei einem an die erste Elektrode grenzenden Kanal mit einem Querschnitt von etwa 10 bis 20 mm² empfehlenswert ist, kann darin bestehen, dass der Durchtrittsquerschnitt etwa 0,8 mm² bis 20 × 10$^{-4}$ mm² beträgt.

Auch ist es empfehlenswert, die Verengung in Strömungsrichtung gesehen nach der ersten Elektrode anzuordnen. Denn hierdurch können sich in den der Verengung vorgeschalteten Strömungskanälen trotz der Zwischenschaltung von Filtern vom Gasstrom noch gegebenenfalls mitgeführte Schmutzteile absetzen, die Gefahr der Verstopfung ist praktisch ausgeschlossen.

Wie das erforderliche Druckverhältnis zwischen der Abströmseite und Zuströmseite erzeugt wird, ist an sich belanglos, weist der Gasstrom jedoch einen Druck auf, der etwa dem Umgebungsdruck gleich ist, z.B. Umgebungsdruck ± 20%, so kann gemäss einer bevorzugten Weiterbildung der Erfindung an die Abströmseite der Verengung eine das erforderliche Druckverhältnis bewirkende Saugvorrichtung angeschlossen sein, die vorzugsweise als Saugpumpe ausgebildet ist. Schwankungen der Saugleistung sind ohne Einfluss auf die Konstanz des Gasstroms solange das kritische Druckverhältnis eingehalten oder unterschritten ist.

Vorteilhaft ist zwischen die Saugvorrichtung und die Abströmseite der Verengung ein Pufferraum eingeschaltet, der Unregelmässigkeiten der Saugvorrichtung glättet. Der Pufferraum weist vorzugsweise ein Volumen auf, das den Gasstrom während 15 bis 60 Minuten aufnehmen könnte.

In Verbindung mit dem Pufferraum kann eine andere Weiterbildung der Erfindung darin bestehen, dass die Saugvorrichtung für diskontinuierlichen Betrieb, insbesondere für Handbetrieb, ausgebildet ist. Hierdurch wird im Pufferraum ein ausreichender Unterdruck erzeugt, der nach der Ausserbetriebnahme der Saugpumpe den Fluss des Gasstromes zumindest für Kurzzeitmessungen aufrechterhält. Um den Druck im Pufferraum und somit das Druckverhältnis erfassen zu können, ist vorteilhaft der Pufferraum mit einem Manometer versehen, gegebenenfalls ist es auch empfehlenswert, an die Zuströmseite und Abströmseite ein Differenzdruckmanometer zur unmittelbaren Erfassung des Druckverhältnisses anzuschliessen.

Weitere Vorteile und empfehlenswerte Merkmale der Erfindung gehen aus der folgenden Beschreibung von Ausführungsbeispielen im Zusammenhang mit den schematischen Zeichnungen hervor. Hierbei zeigen:

Fig. 1 einen Längsschnitt durch den Gegenstand der Erfindung mit einer Verengung in Form einer Düse,

Fig. 2 einen Längsschnitt durch eine Saugpumpe für Handbetrieb, die als Ausführungsvariante an den Pufferraum der Fig. 1 anschliessbar ist,

Fig. 3 einen Längsschnitt durch die Düse gemäss Fig. 1 als Einzelheit und in grösserem Massstab,

Fig. 4 eine Ausführungsvariante des Gegenstands der Fig. 1,

Fig. 5 einen Längsschnitt durch eine Ausführungsvariante der Verengung in Form einer Blende und

Fig. 6 die erfindungsgemässe Messvorrichtung zusammen mit einer Feuerungsanlage.

Gleiche Teile sind in den einzelnen Figuren mit den gleichen Bezugszeichen versehen. Ferner sind in den einzelnen Figuren wiederkehrende Einzelteile nur insoweit mit Bezugszeichen versehen, als dies für das Verständnis erforderlich ist.

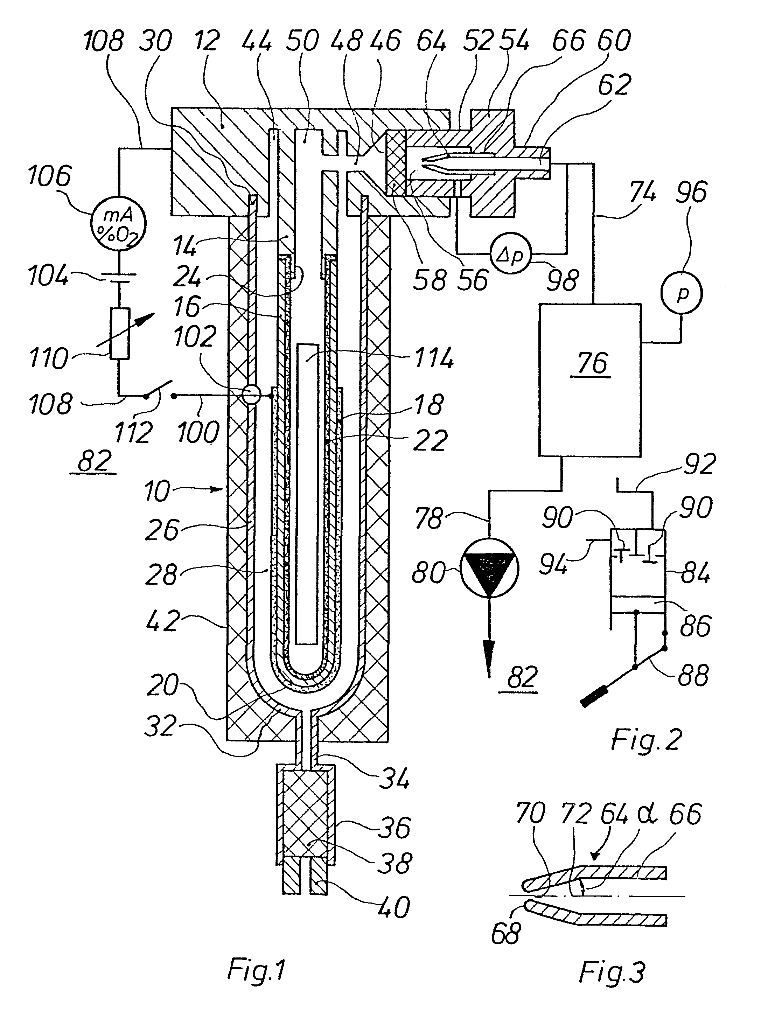

Gemäss Fig. 1 weist die elektrochemische Messvorrichtung eine Messzelle 10 mit einem etwa quaderförmigen Grundkörper 12, zum Beispiel aus Metall, auf. Aus der einen Grundfläche des Grundkörpers 12 ragt ein kreisringzylindrischer Vorsprung 14, an dessen freiem Ende ein Festelektrolytrohr 16 befestigt ist. Das Festelektrolytrohr ist auf seiner Aussenseite mit der ersten Elektrode 18 versehen, die von der verschlossenen Spitze 20 aus gesehen etwa zwei Drittel der Festelektrolytrohrlänge bedeckt. Auf der Innenseite des Festelektrolytrohres 16 ist die zweite Elektrode 22 vorgesehen, die sich von der Spitze 20 bis zur Befestigungsstelle des Festelektrolytrohres 16 am Vorsprung 14 erstreckt und in elektrisch leitendem Kontakt mit dem Vorsprung 14 steht. Dies ist im vorliegenden Ausführungsbeispiel dadurch erreicht, dass die zweite Elektrode 22 mit dem Zentriervorsprung 24 in Berührung steht.

Die Elektroden bestehen aus einem keramischen oder metallischen Material, durch welches der Sauerstoff ungehindert dringen kann, zum Beispiel durch poröse Ausbildung des Materials. Hierfür geeignete Werkstoffe sind zum Beispiel Silber oder Platin, als Werkstoff für das sauerstoffionenleitende Festelektrolytrohr kann Zirkoniumoxid verwendet werden, das mit Kalziumoxid dotiert ist.

Das mit Elektroden versehene Festelektrolytrohr 16 ist von einem Hüllrohr 26 umgeben, so dass zwischen der ersten Elektrode 18 und dem Hüllrohr 26 ein Raum 28 mit kreisringförmigem Querschnitt gebildet ist, wobei die lichte Weite dieses Raumes 28 in radialer Richtung etwa 0,3 bis 2 mm, vorzugsweise etwa 0,5 mm beträgt. Wegen der Übersichtlichkeit ist in den Figuren diese Weite grösser dargestellt. Das Hüllrohr 26 ist in eine kreisringförmige Aussparung 30 des Grundkörpers 12 eingesteckt und befestigt, sein anderes Ende ist kuppenförmig verschlossen, wobei der Abstand der Verschlusskuppe 32 zur ersten Elektrode 18 der ebenfalls kuppenförmigen Spitze 20 etwa gleich ist dem Abstand zwischen dem zylindrischen Bereich des Hüllrohres 26 und der ersten Elektrode 18.

An der Spitze der Verschlusskuppe 32 ist ein Rohrstutzen 34 vorgesehen, der in einer Erweiterung 36 ein Gasfilter 38 aufweist, das zum Beispiel aus Watte oder einem porösen Keramikkörper bestehen kann. Schliesslich ist die Erweiterung 36 mit einem axial durchbohrten Nippel 40 verschlossen, durch welchen das zu messende Gas zugeführt wird. Das gesamte Hüllrohr 26 ist schliesslich noch mit einem Isoliermantel 42 umgeben, der sich bis zum Grundkörper 12 erstreckt. Als Material für den Isoliermantel kann zum Beispiel Mineralwolle eingesetzt werden.

Der am Grundkörper 12 angeordnete ringförmige Vorsprung 14 ist von einem im Grundkörper verlaufenden konzentrischen Ringspalt 44 umgeben, der einerseits in den Raum 28 mündet, und der anderseits an einen quer zur Längsachse der Messzelle verlaufenden, im Querschnitt kreisförmigen Raum 46 angeschlossen ist. Die quer zur Längsachse verlaufende Anschlussbohrung 48 ist hierbei in Richtung zur Längsachse weitergeführt, so dass auch der Innenraum 50 des Vorsprungs 14, welcher mit dem Innenraum des Festelektrolyten eine Einheit bildet, an den Raum 46 angeschlossen ist.

Der Raum 46, der vorzugsweise als Bohrung ausgebildet ist, weist Gewinde auf, in welches ein Einsatz 52 mit Hilfe eines am Einsatz vorgesehenen Kopfes 54 eingeschraubt ist. Der in den Grundkörper 12 ragende Bereich des Einsatzes 52 ist kreisringförmig ausgebildet und klemmt ein scheibenförmiges Filter 58 zwischen sich und einer kegelförmigen Verjüngung des Raumes 46 ein. Die Verjüngung bildet hierbei den Übergang des Raumes 46 zur Anschlussbohrung 48, das Filter 58 ist bezüglich seines Materials identisch mit dem Gasfilter 38.

Am Kopf 54 ist ein nach aussen ragender Anschlussnippel 60 zentrisch angeordnet, der von einem zentrisch verlaufenden und zur Ausnehmung 56 führenden Kanal 62 durchdrungen ist. Im Bereich seiner inneren Ausmündung weist der Kanal 62 eine Stufe auf, in welcher die Düse 64 mit ihrem zylindrischen Bereich 66 befestigt ist, wobei die Düse 64 frei in die Ausnehmung 56 ragt.

Der Aufbau der Düse ist am besten aus Fig. 3 zu ersehen, welche die Düse als Einzelheit und im grösseren Massstab darstellt. Danach ist der Einlauf 68 der Düse abgerundet und führt zum engsten Durchtrittsquerschnitt 70, an den sich eine kegelförmige Erweiterung 72 anschliesst, wobei der Öffnungswinkel $\alpha$ der Erweiterung etwa 8 bis 18°, vorzugsweise 10 bis 15° beträgt. An die Erweiterung 72 schliesst sich der zylindrische Bereich 66 an.

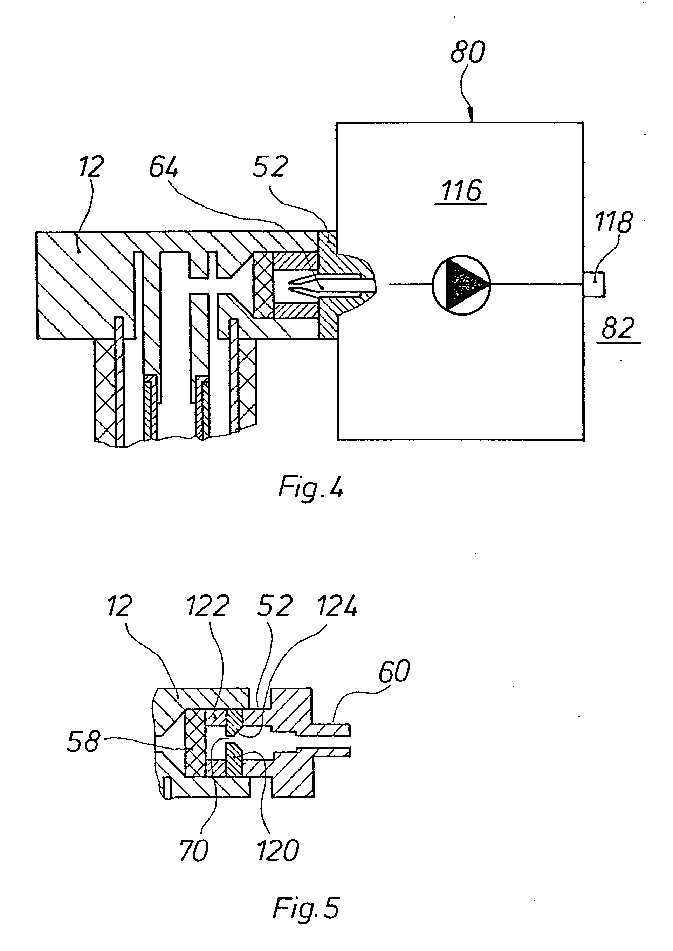

Die engste Stelle des Durchtrittsquerschnitts 70 ist nun so gewählt, dass bei einer Durchströmung dieser Stelle mit Schallgeschwindigkeit eine ausreichende Gasmenge durchtritt. Als Richtwert für den Durchtrittsquerschnitt 70 kann im vorliegenden Ausführungsbeispiel, bei dem der Raum 28 einen Querschnitt von etwa 10 bis 20 mm² aufweist, ein Wert von etwa 0,8 bis 20 × $10^{-4}$ mm² gelten. Die Gesamtlänge der Düse ist etwa 15 bis 30 mm, die Länge der Erweiterung 72 liegt zwischen 5 und 10 mm bei einer Lichtweite des zylindrischen Bereichs 66 von etwa 5 bis 8 mm. Als bevorzugtes Material für die Düse dient Glas, auch mit Laserstrahlen durchbohrte Saphire sind empfehlenswert. Der gasführende Querschnitt der Düse ist kreisförmig, das Gleiche gilt auch für die Blende 120 in Fig. 5.

Wie aus Fig. 1 weiter zu ersehen, verbindet die Düse die Ausnehmung 56, welche die Zuströmseite des Gases bildet, mit dem Kanal 62, welcher die Abströmseite der Düse darstellt.

An den Anschlussnippel 60 ist über eine Anschlussleitung 74 ein Pufferraum 76 angeschlossen, der über eine weitere Leitung 78 mit einer Saugvorrichtung 80, zum Beispiel in Form einer Gaspumpe, verbunden ist, deren Auslass in den Aussenraum 82, oder insbesondere bei giftigen Gasen in einen Auffangraum mündet.

In Fig. 2 ist eine Ausführungsvariante bezüglich der Saugvorrichtung dargestellt, die anstelle der Saugvorrichtung 80 an den Pufferraum 76 angeschlossen werden kann. Die Saugvorrichtung gemäss Fig. 2 besteht aus einer Handpumpe 84, deren Kolben 86 durch einen Pumpenhebel 88 betätigt werden kann, so dass über die angedeuteten Ventile 90 Gas aus dem Pufferraum 76 über die Leitung 92 angesaugt und durch den Auslass 94 in den Aussenraum ausgestossen werden kann.

Zum Erfassen der Druckverhältnisse des Gasstroms ist am Pufferraum 76 ein Manometer 96 angeordnet, oder gegebenenfalls kann ein Differenzmanometer 98 zwischen die Ausnehmung 56 und die Anschlussleitung 74 oder den Kanal 62 eingeschaltet sein.

Um an die Elektroden 18, 22 die erforderliche Messspannung legen zu können, führt von der ersten Elektrode 18 eine elektrische Leitung 100 radial in den Aussenraum 82, wobei an der Durchdringungsstelle des Hüllrohres 26 eine Isolierperle 102 eingefügt ist. Die zweite Elektrode 22 ist über den Zentriervorsprung 24 und den Vorsprung 14 mit dem Grundkörper 12 elektrisch leitend verbunden, so dass der Grundkörper 12 zusammen mit der elektrischen Leitung 100 als elektrische Anschlüsse der Elektroden dienen. Diesen Anschlüssen wird die für die Messung erforderliche Spannung über elektrische Leitungen 108 von einer Gleichspannungsquelle 104, zum Beispiel in Form einer elektrischen Batterie, zugeführt, wobei ein Strommessgerät 106, zum Beispiel ein Milliamperemeter, ein Regelwiderstand 110 und ein elektrischer Schalter 112 eingefügt sind.

Im Innenraum des Festelektrolytrohres 16 ist noch eine, vorzugsweise elektrische Heizung 114 angeordnet, um das Festelektrolytrohr auf Betriebstemperatur zu bringen. Die zur Heizung 114 führenden elektrischen Anschlussleitungen sind in Fig. 1 nicht dargestellt.

Bevor mit der vorbeschriebenen Messvorrichtung Sauerstoffmessungen durchgeführt werden können, ist eine Ersteinstellung bzw. Eichung erforderlich, die am besten gleich bei der Herstellung der Messvorrichtung durchgeführt wird. Hierzu ist es am einfachsten, ein Gas zu benutzen, das in chemischer und physikalischer Hinsicht dem zu messenden Gas ähnlich ist. Soll die Messvorrichtung für die Bestimmung des Sauerstoffgehaltes in Abgasen eingesetzt werden, so kann für die Eichung Luft benutzt werden. Das kritische Druckverhältnis für Luft als zweiatomiges Gas beträgt hierbei 0,528.

Für die Ersteinstellung wird zunächst die Heizung 114 und dann die Saugvorrichtung 80 in Betrieb genommen und deren Saugleistung so eingestellt, dass das Druckverhältnis zwischen der Abströmseite 62 und Zuströmseite 56 der Düse 64 gleich dem kritischen Druckverhältnis oder vorzugsweise kleiner ist. Die Einstellung geschieht hierbei mit Hilfe des Differenzdruckmanometers 98. Ist der Druck des zugeführten Eichgases bekannt, zum Beispiel beim Ansaugen von Umgebungsluft, so genügt auch die Messung des Druckes auf der Abströmseite der Düse, zum Beispiel des Druckes im Pufferraum 76 für die Bestimmung des Druckverhältnisses. Ist die Ersteinstellung der Saugvorrichtung 80 einmal auf diese Weise durchgeführt worden, so ist eine weitere laufende Überwachung des Druckverhältnisses nicht erforderlich, wenn diese Einstellung beibehalten wird. Ein Gasstrom von gleichbleibender Grösse ist somit auch im Langzeitbetrieb gewährleistet.

Für den Fall, dass das zu messende Gas der Messvorrichtung mit Überdruck zugeführt wird, sind eine Saugvorrichtung 80 und gegebenenfalls ein Pufferraum 76 nicht erforderlich, falls die Abströmseite 62 der Düse auf niedrigerem Druck liegt, zum Beispiel durch Verbindung mit dem Aussenraum, und sich das gewünschte Druckverhältnis somit einstellt.

Ist nun auf vorbeschriebene Weise die Ersteinstellung durchgeführt worden und der Festelektrolyt auf Betriebstemperatur gebracht, so wird der Schalter 112 geschlossen und an die Elektroden 18, 22 eine Gleichspannung von etwa 1 Volt gelegt. Die Regulierung dieser Spannung kann durch den Regelwiderstand 110 erfolgen. Der Anzeige des Strommessgerätes 106 wird jetzt ein Sauerstoffgehalt von 21 Volumenprozent zugeordnet. Da beim vorliegenden Messprinzip ein linearer Zusammenhang zwischen dem Sauerstoffgehalt des zu messenden Gases und dem im Messkreis fliessenden Strom besteht, genügt ein einziger Messpunkt für die Eichung.

Soll mit der Messvorrichtung jetzt der Sauerstoffgehalt von Verbrennungsabgasen gemessen werden, so ist die Messvorrichtung mit Hilfe des Nippels 40 und gegebenenfalls unter Zwischenschaltung eines Gaskühlers mit der Abgasleitung zu verbinden. Die Saugvorrichtung 80 saugt jetzt einen konstanten Gasstrom auf folgendem Weg durch die Messvorrichtung: Gasfilter 38, Raum 28, Anschlussbohrung 48, Raum 46, Filter 58, Ausnehmung 56, welche gleichbedeutend ist mit der Zuströmseite der Düse, Düse 64, Kanal 62, welcher gleichbedeutend ist mit der Abströmseite der Düse, Pufferraum 76 und Saugvorrichtung 80. Beim Vorbeiströmen des Gasstromes an der ersten Elektrode 18 wird der im Gasstrom enthaltene Sauerstoff extrahiert, in Form von Ionen durch den Festelektrolyten geleitet und an der zweiten Elektrode 22 wiederum zu Sauerstoff oxidiert und in den Innenraum des Festelektrolytrohres abgegeben. Der Sauerstoff strömt dann durch die im Vorsprung 14 vorgesehene radiale Anschlussbohrung 48 ab und wird dem Gasstrom wieder beigemischt. Der hierbei von der Gleichspannungsquelle 104 bei geschlossenem Schalter 112 über die Elektroden 18, 22 fliessende Strom wird hierbei vom Strommessgerät 106 erfasst. Dieser Strom ist ein Mass für den Sauerstoffgehalt des Gases, gegebenenfalls erfolgt die Anzeige des Strommessgerätes unmittelbar in Volumenprozent Sauerstoff.

In den meisten Fällen wird die Saugvorrichtung 80 als elektrische Saugpumpe ausgebildet sein. Für Kurzzeitmessungen, zum Beispiel für Betriebsüberwachungen, ist es jedoch empfehlenswert, anstelle der Saugvorrichtung 80 eine Handpumpe 84 einzusetzen. Dieser Fall ist in Fig. 2 dargestellt. Mit Hilfe der Handpumpe 84, die an den Pufferraum 76 angeschlossen ist, wird im Pufferraum ein für die Erzeugung des kritischen Druckverhältnisses ausreichender Unterdruck erzeugt und durch die Wirkung des Pufferraums konstant gehalten. Wird hierbei der für die Erzeugung des kritischen Druckverhältnisses erforderliche Unterdruck im Pufferraum 76 stark unterschritten, so ist ein intermittierender Betrieb der Handpumpe 84 möglich, denn in den Betriebspausen der Pumpe sichert der Unterdruck des Pufferraums 76 den für die Messung erforderlichen konstanten Gasfluss.

In Fig. 4 ist eine Ausführungsvariante des Gegenstands der Fig. 1 bezüglich der Saugvorrichtung 80 dargestellt. Während bei der Messvorrichtung nach Fig. 1 die Saugvorrichtung 80 aus einem getrennten Bauteil besteht, ist bei der Ausführungsvariante gemäss Fig. 4 die Saugvorrichtung 80 unmittelbar am Grundkörper 12 befestigt. Die Saugvorrichtung 80 ist hierzu vorzugsweise als elektrische Saugpumpe 116 ausgebildet und unmittelbar am Einsatz 52 vorzugsweise lösbar befestigt. Der Auslass 118 der Saugpumpe mündet hierbei in den Aussenraum 82. Die vorliegende Ausführungsform weist gegenüber Fig. 1 einen kompakteren Aufbau auf.

In Fig. 5 ist der Bereich des Einsatzes 52 als Einzelheit und als Ausführungsvariante dargestellt. Die in den Weg des Gasstroms eingefügte Verengung ist hier als Blende 120 ausgebildet. Diese besteht aus einer kreisringförmigen Scheibe, die zwischen dem Einsatz 52 und einem sich am Filter 58 abstützenden Zwischenring 122 eingeklemmt ist. Die Blende 120 ist mit einer vorzugsweise zentrischen Öffnung versehen, welche den Durchtrittsquerschnitt 70 bildet. Um die Reibungsverluste bei der Durchströmung des Durchtrittsquerschnitts 70 gering zu halten, weist die Blende 120 an der Abströmseite eine kegelförmige Erweiterung 124 des zylindrischen Durchtrittsquerschnitts auf. Die Dicke der Blende beträgt etwa 2 bis 5 mm, die Erweiterung 124 durchdringt ungefähr $^2/_3$ der Blendendicke. Als Material für die Blende ist vorzugsweise Glas vorgesehen.

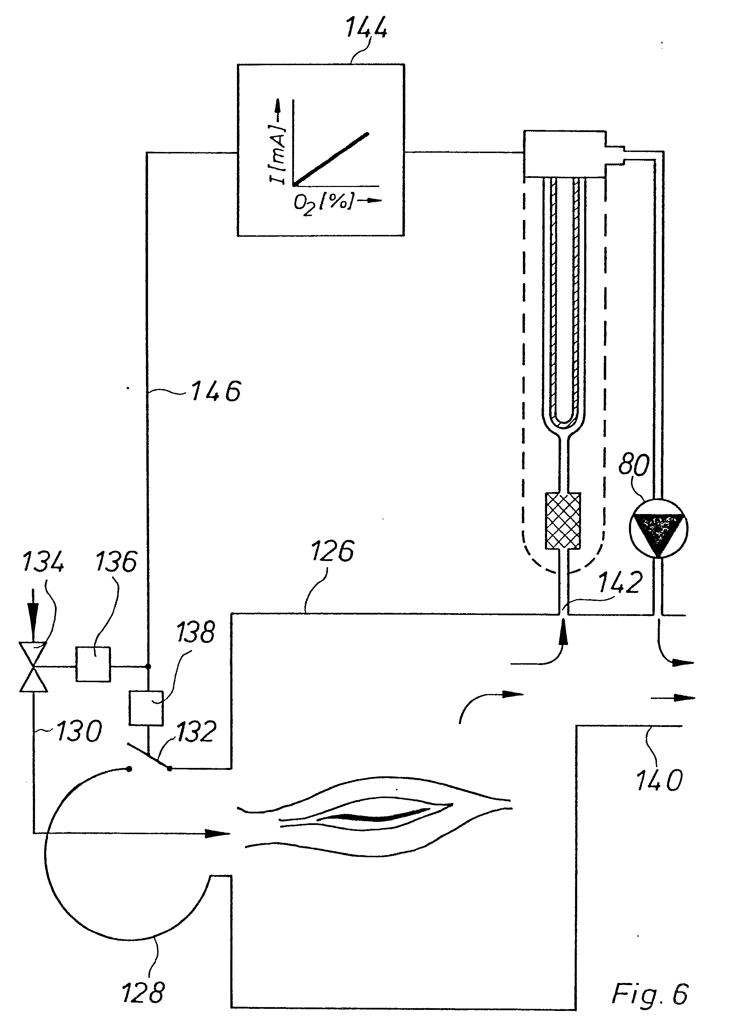

In Fig. 6 ist eine bevorzugte Anwendung der erfindungsgemässen Messvorrichtung zur Verbrennungsregelung von Feuerungen dargestellt. Ein Heizkessel 126 ist mit einem Brenner 128 für strömende Brennstoffe wie Gas oder Öl ausgerüstet. Der Brennstoff wird hierbei über die Rohrleitung 130, die Verbrennungsluft über die Luftklappe 132 zugeführt. In der Rohrleitung 130 ist ein Regelventil 134 mit einer Stellvorrichtung 136 vorgesehen, wogegen die Luftklappe 132 mit einer Stellvorrichtung 138 versehen ist. An den Abgaskanal 140, durch welchen die Verbrennungsabgase zum Kamin abströmen, ist die erfindungsgemässe Messvorrichtung angeschlossen, wobei der von der Saugvorrichtung 80 aus dem Abgaskanal 140 durch die Messvorrichtung gesaugte Gasstrom stromabwärts der Entnahmestelle 142 wieder in den Abgaskanal 140 geleitet wird. Die Saugvorrichtung ist im vorliegenden Ausführungsbeispiel als kontinuierlich arbeitende elektrische Saugpumpe ausgebildet, so dass auf einen Pufferraum verzichtet werden kann.

Anstelle eines Strommessgerätes oder zusätzlich ist im vorliegenden Fall ein Regelgerät 144 über elektrische Leitungen mit der Messvorrichtung verbunden. Im schematisch angedeuteten Regelgerät 144 ist der lineare Zusammenhang zwischen Sauerstoffgehalt des Messgases und dem von der Messvorrichtung abgegebenen Strom in Milliampere angedeutet. Der Ausgang des Regelgerätes 144 steht zum Beispiel über elektrische Leitungen 146 in Wirkverbindung mit den Stellvorrichtungen 136 und 138.

Während des Betriebs des Brenners 128 werden von der Saugvorrichtung 80 Verbrennungsabgase durch die Messvorrichtung gesaugt und deren Sauerstoffgehalt erfasst. Da bei einer wirtschaftlichen Verbrennung die Verbrennungsabgase möglichst wenig Sauerstoff enthalten sollen, werden im Regelgerät 144 von den in der Messvorrichtung erfassten Sauerstoffsignalen Regelbefehle abgeleitet, die über die Leitung 146 der auf die Luftklappe 132 einwirkenden Stellvorrichtung 138 und/oder jener Stellvorrichtung 136 zugeführt werden, welche auf das Regelventil 134 einwirkt. Das Regelgerät 144 ist nun so programmiert, dass die Verbrennungsluftzufuhr zum Brenner durch Verstellung der Luftklappe 132 so eingeregelt wird, dass möglichst geringer Sauerstoffgehalt in den Abgasen vorhanden ist. Gegebenenfalls kann auch noch die Brennstoffzufuhr über die Verstellvorrichtung 136 beeinflusst werden, falls die Beeinflussung der Luftklappe 132 nicht ausreichen sollte. Auf diese Weise ist unter allen Betriebsbedingungen eine optimale Einstellung des Brenners 128 und somit einwandfreie, wirtschaftliche Verbrennung gewährleistet.

Anstelle einer Anordnung der Saugvorrichtung 80 auf der Abströmseite der Düse ist es ebensogut möglich, eine Druckvorrichtung, zum Beispiel in Form einer Druckpumpe, in jener Leitung anzuordnen, welche an den Nippel 40 angeschlossen ist und das Gas zur Messvorrichtung leitet (vgl. Fig. 1).

Bezüglich der Heizung 114 ist noch zu bemerken, dass für den Betrieb der Messvorrichtung keine konstante und auch keine bestimmte Temperatur erforderlich ist. Das Festelektrolytrohr 16 ist lediglich auf eine Temperatur aufzuheizen, die oberhalb einer Grenztemperatur von etwa 400 °C liegt.

**Patentansprüche**

1. Messvorrichtung zur Bestimmung des Sauerstoffgehaltes in Gasen von etwa gleichbleibendem Druck, insbesondere in Verbrennungsabgasen, mit einer elektrochemischen Messzelle, die einen sauerstoffionenleitenden Festelektrolyten (16) aufweist, der mit zwei Elektroden (18, 22) versehen ist, mit einer Einrichtung zur Erzeugung eines die eine Elektrode (18) beaufschlagenden vorgegebenen konstanten Stromes des zu überwachenden Gases und einer zwischen die Elektroden (18, 22) schaltbaren Reihenschaltung aus einer elektrischen Gleichstromquelle (104) und einem Strommessgerät (106), dadurch gekennzeichnet, dass zur Konstanthaltung des zu überwachenden Gasstromes im Wege dieses Gasstromes wenigstens eine Verengung (64, 120) angeordnet ist, deren Durchtrittsquerschnitt bei Schallgeschwindigkeit des Gases auf die Grösse des Stromes abgestimmt ist, und dass Mittel (80, 84) zur Einstellung des Druckverhältnisses zwischen Abströmseite (62) und Zuströmseite (56) der Verengung (64, 120) auf einen Wert vorgesehen sind, bei dem das Gas mit Schallgeschwindigkeit durch die Verengung strömt.

2. Messvorrichtung nach Anspruch 1, dadurch gekennzeichnet, dass die Verengung durch eine Blende (120) gebildet ist.

3. Messvorrichtung nach Anspruch 1, dadurch gekennzeichnet, dass die Verengung durch eine Düse (64) gebildet ist.

4. Messvorrichtung nach Anspruch 2 oder 3, dadurch gekennzeichnet, dass die Blende (120) bzw. die Düse (64) aus Glas gefertigt ist.

5. Messvorrichtung nach einem der Ansprüche 1 bis 4, dadurch gekennzeichnet, dass bei einem an die eine Elektrode (18) angrenzenden Raum (28) für den Gasstrom mit einem Querschnitt von etwa 10 bis 20 mm² der Durchtrittsquerschnitt (70) der Verengung an seiner engsten Stelle etwa 0,8 bis 20 × $10^{-4}$ mm² beträgt.

6. Messvorrichtung nach einem der Ansprüche 1 bis 5, dadurch gekennzeichnet, dass die Verengung (64, 120) in Strömungsrichtung gesehen nach der einen Elektrode (18) angeordnet ist.

7. Messvorrichtung nach einem der Ansprüche 1 bis 6, dadurch gekennzeichnet, dass an die Abströmseite (62) eine das erforderliche Druckverhältnis bewirkende Saugvorrichtung (80) angeschlossen ist.

8. Messvorrichtung nach Anspruch 7, dadurch gekennzeichnet, dass die Saugvorrichtung (80) eine Saugpumpe ist.

9. Messvorrichtung nach Anspruch 7 oder 8, dadurch gekennzeichnet, dass zwischen die Saugvorrichtung (80) und die Abströmseite (62) ein Pufferraum (76) eingeschaltet ist.

10. Messvorrichtung nach Anspruch 8 und 9, dadurch gekennzeichnet, dass die Saugpumpe für diskontinuierlichen Betrieb, insbesondere als Handpumpe (84), ausgebildet ist.

11. Verwendung einer Messvorrichtung nach einem der Ansprüche 1 bis 9 als Messwertgeber einer Anlage zur Regelung des Brennstoff-Luftverhältnisses einer Feuerung für strömende Brennstoffe.

**Claims**

1. Measuring instrument for determining the oxygen content in gases of approximately constant pressure, in particular in combustion off-gases, with an electrochemical measuring cell having a solid electrolyte (16) which conducts oxygen ions and is provided with two electrodes (18, 22), with a device for generating a predetermined constant stream, applied to one electrode (18), of the gas to be monitored and a series circuit which can be connected between the electrodes (18, 22) and consists of an electrical direct-current source (104) and a current-measuring instrument (106), characterised in that, for keeping constant the gas stream which is to be monitored, at least one restriction (64, 120), the passage cross-section of which at the velocity of sound of the gas is matched to the magnitude of the current, is provided in the path of this geyser stream, and that means (80, 84) are provided for adjusting the pressure ratio between the downstream side (62) and the upstream side (56) of the restriction (64, 120) to a value at which the gas flows through the restriction at the velocity of sound.

2. Measuring instrument according to Claim 1, characterised in that the restriction is formed by an orifice plate (120).

3. Measuring instrument according to Claim 1, characterised in that the restriction is formed by a nozzle (64).

4. Measuring instrument according to Claim 2 or 3, characterised in that the orifice plate (120) or the nozzle (64) is made of glass.

5. Measuring instrument according to one of Claims 1 to 4, characterised in that, in the case of a chamber (28) for the gas stream, adjoining the first electrode (18) and having a cross-section of about 10 to 20 mm², the passage cross-section (70) of the restriction is about 0.8 to 20 × $10^{-4}$ mm² at its narrowest point.

6. Measuring instrument according to one of Claims 1 to 5, characterised in that the restriction (64, 120) is arranged after the first electrode (18), as viewed in the direction of flow.

7. Measuring instrument according to one of Claims 1 to 6, characterised in that a suction device (80) producing the requisite pressure ratio is connected to the downstream side (62).

8. Measuring instrument according to Claim 7, characterised in that the suction device (80) is a suction pump.

9. Measuring instrument according to Claim 7 or 8, characterised in that a buffer chamber (76) is interposed between the suction device (80) and the downstream side (62).

10. Measuring instrument according to Claim 8 or 9, characterised in that the suction pump is designed for discontinuous operation, in particular as a hand pump (84).

11. Use of a measuring instrument according to one of Claims 1 to 9, as the sensing element of a unit for controlling the fuel/air ratio in a furnace with fuel flow.

### Revendications

1. Dispositif de mesure pour déterminer la concentration en oxygène dans des gaz ayant une pression demeurant à peu près égale, en particulier des gaz de combustion, comportant une cellule de mesure électrochimique qui présente un électrolyte solide (16) conduisant les ions oxygène et qui est muni de deux électrodes (18, 22), un dispositif pour produire un courant prédéterminé constant, alimentant l'une des électrodes (18), du gaz à surveiller, et un circuit série commutable entre les électrodes (18, 22) et composé d'une source de courant continu électrique (104) et d'un appareil de mesure de courant (106), caractérisé en ce que pour maintenir constant le courant de gaz à surveiller, on dispose dans le trajet de ce courant de gaz au moins un rétrécissement (64, 120) dont la section transversale de passage est réglée à la vitesse sonique du gaz sur l'amplitude du courant, et en ce que des moyens (80, 84) sont prévus pour régler le rapport de pressions entre le côté de sortie d'écoulement (62) et le côté d'arrivée d'écoulement (56) du rétrécissement (64, 120) à une valeur pour laquelle le gaz s'écoule à travers le rétrécissement avec la vitesse du son.

2. Dispositif de mesure selon la revendication 1, caractérisé en ce que le rétrécissement est formé par un diaphragme (120).

3. Dispositif de mesure selon la revendication 1, caractérisé en ce que le rétrécissement est formé par une buse (64).

4. Dispositif de mesure selon la revendication 2 ou 3, caractérisé en ce que le diaphragme (120) ou la buse (64) sont fabriqués en verre.

5. Dispositif de mesure selon l'une des revendications 1 à 4, caractérisé en ce que pour un espace (28) destiné au courant de gaz avoisinant l'une des électrodes (18) et ayant une section transversale d'environ 10 à 20 mm², la section transversale de passage (70) du rétrécissement à son point le plus étroit est d'environ 0,8 à 20 × $10^{-4}$ mm².

6. Dispositif de mesure selon l'une des revendications 1 à 5, caractérisé en ce que le rétrécissement (64, 120) vu dans la direction d'écoulement est disposé après l'une des électrodes (18).

7. Dispositif de mesure selon l'une des revendications 1 à 6, caractérisé en ce que sur le côté de sortie d'écoulement (62) on raccorde le dispositif d'aspiration (80) provoquant le rapport nécessaire des pressions.

8. Dispositif de mesure selon la revendication 7, caractérisé en ce que le dispositif d'aspiration (80) est une pompe aspirante.

9. Dispositif de mesure selon la revendication 7 ou 8, caractérisé en ce que, entre le dispositif d'aspiration (80) et le côté de sortie d'écoulement (62), on introduit un espace tampon (76).

10. Dispositif de mesure selon la revendication 8 et la revendication 9, caractérisé en ce que la pompe aspirante est conformée pour un fonctionnement discontinu, en particulier en tant que pompe manuelle (84).

11. Utilisation d'un dispositif de mesure selon l'une des revendications 1 à 9 en tant que générateur de valeur de mesure d'une installation pour réguler le rapport de carburant et d'air d'une combustion avec des carburants qui s'écoulent.

Fig.1

Fig.2

Fig.3

Fig.4

Fig.5

0 028 826

*Fig. 6*